Europäisches Patentamt

European Patent Office

Office européen des brevets

(19)

(11) Numéro de publication : **0 224 496**
**B1**

(12) **FASCICULE DE BREVET EUROPÉEN**

(45) Date de publication du fascicule du brevet :
**24.01.90**

(21) Numéro de dépôt : **86902422.4**

(22) Date de dépôt : **16.04.86**

(86) Numéro de dépôt international :
**PCT/FR 86/00127**

(87) Numéro de publication internationale :
**WO/8606094 (23.10.86 Gazette 86/23)**

(51) Int. Cl.⁵ : **C 12 M   3/00, C 12 M   1/12**

(54) **ENSEMBLE COMPOSITE DE MEMBRANES DE FILTRATION.**

(30) Priorité : **17.04.85 FR 8505766**

(43) Date de publication de la demande :
**10.06.87 Bulletin 87/24**

(45) Mention de la délivrance du brevet :
**24.01.90 Bulletin 90/04**

(84) Etats contractants désignés :
**AT BE CH DE FR GB IT LI LU NL SE**

(56) Documents cités :
**EP—A— 0 112 155**
**EP—A— 0 113 328**
**FR—A— 2 236 004**
**FR—A— 2 393 849**

(73) Titulaire : **LYONNAISE DES EAUX Société Anonyme**
**52 rue de Lisbonne**
**F-75008 Paris (FR)**

(72) Inventeur : **BERSILLON, Jean-Luc**
**1bis, rue du Printemps**
**F-78230 Le Pecq (FR)**

(74) Mandataire : **Cournarie, Michèle et al**
**OFFICE BLETRY R.& G. ROGER-PETIT & R.BLETRY 2,**
**boulevard de Strasbourg**
**F-75010 Paris (FR)**

Jouve, 18, rue St-Denis, 75001 Paris, France

**Description**

L'invention concerne un ensemble composite ordonné de membranes de filtration permettant une réaction biologique (ou fermentation) et une filtration simultanée des produits de la réaction, et son utilisation dans un bioréacteur.

Les réactions biologiques ou fermentations sont des processus largement utilisés dans de nombreux domaines : industries alimentaires (production du vin, de la bière, etc...), industries pharmaceutiques (production des antibiotiques), traitements industriels des eaux, etc...

Une fois la fermentation terminée, se pose le problème de la séparation des produits de la réaction, en produits recherchés et en produits à rejeter. En règle générale, les produits recherchés sont en solution dans l'eau qui contient en suspension de nombreuses impuretés. Les techniques classiques de séparation sont la filtration et la décantation.

L'usage des membranes de micro filtration et d'ultrafiltration se répand de plus en plus et on a déjà émis l'idée d'associer un réacteur biologique ou bioréacteur à des membranes de filtration. Toutefois cette association est limitée en ce que la filtration s'effectue à la sortie du bioréacteur, le produit de la réaction étant généralement introduit dans un faisceau de fibres creuses constituant les membranes de filtration qui effectuent la séparation désirée.

D'autre part, des membranes sous forme de fibres creuses ont été utilisées pour la culture in vitro de cellules (voir US-A-3 883 393, US-A-3 821 087 et US-A-4 200 689 ; ainsi que US-A-4 201 845, US-A-3 997 386 et US-A-4 220 725).

Le document EP-A-0 113 328 décrit un dispositif de culture de cellules animales in vitro dans lequel les cellules sont retenues dans une matrice rigide alimentée en milieu nutritif à travers un tube de porosité relativement faible tandis que les produits cellulaires et le milieu nutritif épuisé sont extraits à travers un tube de porosité relativement élevée, une alimentation en oxygène étant prévue grâce à une membrane tubulaire sélectivement perméable. Les différents éléments sont disposés coaxialement et constituent un réacteur.

On a cherché à mettre au point un réacteur biologique utilisant des membranes de filtration, pour effectuer simultanément ou pratiquement simultanément une réaction biologique et la séparation des produits obtenus.

Pour ce faire, il a été conçu un ensemble composite ordonné de membranes de filtration, comprenant :

- une première membrane constituée d'une fibre creuse ou d'un faisceau de fibres creuses amenant le substrat à traiter et laissant passer de façon centrifuge les produits sur lesquels doit se faire la réaction biologique,

- une seconde membrane discontinue et constituée d'une série de fibres de perméation gazeuse, disposées autour de la première membrane, cette seconde membrane assurant les échanges gazeux de la réaction biologique, et

- une troisième membrane discontinue et constituée d'une série de fibres creuses, situées au voisinage des fibres de perméation gazeuse de la seconde membrane et laissant passer de façon centripète les produits recherchés de la réaction, les membranes étant séparées par des écarteurs qui permettent le passage des différents flux entre les membranes et les pressions de ces différents flux étant choisies de façon appropriée.

Deux types principaux d'ensemble sont envisageables, l'un pour une fermentation aérobie, l'autre pour une fermentation anaérobie. Dans le premier cas, les fibres de perméation gazeuse de la deuxième membrane ont pour rôle d'amener l'air ou de l'oxygène au voisinage des microorganismes, tandis que dans l'autre cas, les fibres de perméation gazeuse permettent l'enlèvement des gaz produits par la fermentation anaérobie.

Un tel ensemble de membranes de filtration peut être utilisé, seul ou avec d'autres ensembles similaires, dans un réacteur biologique comportant de façon classique une arrivée de substrat à traiter, éventuellement une arrivée de gaz, une sortie d'effluent gazeux, une sortie d'effluent de produits désirés et un circuit de recyclage du substrat non traité ; un circuit de recyclage interne de l'effluent traité est également prévu et son rôle sera décrit de façon détaillée ci-après.

Ceci permet la mise en oeuvre d'un procédé de traitement biologique et filtration simultanée dans lequel on amène un substrat à traiter sur des microorganismes fixés et on filtre le produit fermenté pour séparer les produits recherchés de l'effluent à rejeter, dans lequel on introduit le substrat à traiter dans un réacteur contenant un ensemble ou un faisceau d'ensembles composites de membranes de filtration, le substrat arrivant à l'intérieur de la fibre creuse constituant la première membrane, on établit une culture de microorganismes fixés au voisinage des fibres de perméation gazeuse, et on recueille les produits désirés par l'intermédiaire des fibres constituant la troisième membrane.

Le fait que les deuxième et troisième membranes soient discontinues permet de les disposer comme on le souhaite par rapport à la première membrane. Comme certaines dispositions, par exemple selon une disposition hexagonale centrée comme décrit ci-dessous ou selon d'autres formes à base de carré, losange ou polygones, permettent un « pavage » régulier, on voit qu'il est possible d'augmenter la section interne du réacteur, et donc sa capacité de fonctionnement et de traitement. Au contraire, pour les réacteurs de la technique antérieure, on ne peut augmenter leur capacité qu'en augmentant le diamètre des membranes coaxiales, ce qui modifie les paramètres de la réaction biologique.

L'invention va maintenant être décrite avec référence aux dessins annexés dans lesquels :

- la figure 1 est une représentation schématique

d'une première forme de réalisation d'un ensemble composite de membranes de filtration selon l'invention,

- la figure 2 est une représentation schématique d'une seconde forme de réalisation d'un ensemble selon l'invention,

- la figure 3 représente un faisceau d'ensembles composites,

- la figure 4 est le schéma de principe du fonctionnement d'un bioréacteur utilisant les ensembles de filtration de la présente invention.

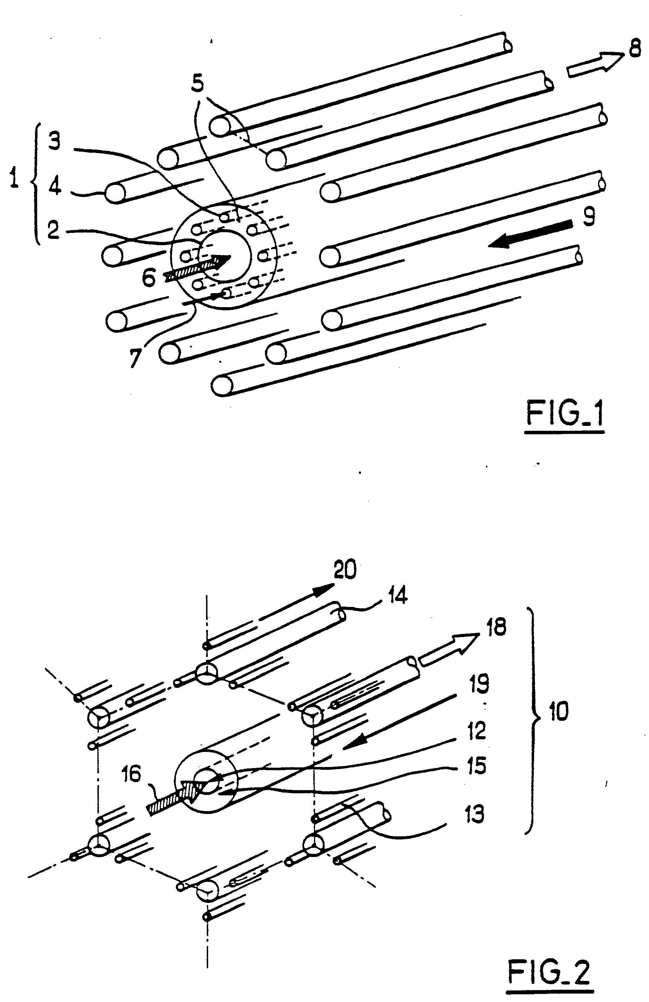

La figure 1 représente une première forme de réalisation d'un ensemble composite selon l'invention. Cet ensemble 1 se compose d'une première membrane de filtration 2, constituée d'une fibre creuse de diamètre relativement important. Autour de cette fibre 2, est disposée une série de fibres 3 de perméation gazeuse qui constitue la deuxième membrane. Ces fibres sont réparties régulièrement autour de la fibre 2, à l'aide d'un écarteur 5, de façon concentrique par exemple, et leur nombre est fonction de la dimension relative des deux types de fibres et des paramètres de la réaction biologique. La troisième membrane est constituée par une série de fibres 4 disposées de façon régulière autour des fibres. Des écarteurs 5 permettent de maintenir en place les différentes séries de fibres autour de la fibre centrale 2. Sur la figure 1, il a été représenté huit fibres creuses 3 et douze fibres creuses 4, les fibres 3 étant réparties de façon concentrique autour de la fibre 2 alors que les fibres 4 occupent les sommets et le milieu des côtés d'un hexagone entourant la série de fibres 3. Il est entendu que cette disposition peut être modifiée et que le nombre et la disposition relative des trois types de fibres ne sont pas limités à ceux représentés de façon particulière sur la figure 1.

Les fibres de filtration sont des produits connus en soi et l'on peut utiliser celles qui sont disponibles à l'heure actuelle en les choisissant en fonction des caractéristiques que l'on désire. De même, les écarteurs 5 sont constitués de treillis ou de mousses poreuses permettant la circulation des différents flux entre les trois types de membranes.

Le fonctionnement de cet ensemble composite va maintenant être décrit en détail.

Le substrat à traiter, c'est-à-dire de façon générale une suspension de produits divers dans de l'eau contenant également en solution des produits de nature variable, doit subir une réaction biologique (ou fermentation) à l'aide d'un microorganisme ou d'un ensemble de microorganismes effectuant une ou une série de réactions précises. On citera à titre d'exemples la fermentation alcoolique, la production d'antibiotiques, la méthanisation, la nitrification, etc.... La réaction peut se faire de façon aérobie ou de façon anaérobie. Dans le cas d'une réaction aérobie, le rôle des fibres de perméation gazeuse est d'amener de l'air ou de l'oxygène aux microorganismes. Dans le cas d'une fermentation anérobie, le rôle des fibres de perméation gazeuse est d'éliminer les gaz produits par le métabolisme des microorganismes, qui peuvent gêner la poursuite de la réaction ou même constituer des poisons pour les microorganismes. La suite de la description de la figure 1 sera faite en considérant que l'on effectue ici une fermentation aérobie (avec apport d'air, par exemple).

Le substrat à traiter 6 circule à l'intérieur de la fibre creuse 2 qui laisse passer vers l'extérieur les produits devant subir la réaction biologique. Ces produits se trouvent alors au voisinage des fibres de perméation gazeuse par lesquelles arrive l'air 7 nécessaire à la vie des microorganismes. Les microorganismes introduits au préalable dans le système se rassemblent là où ils trouvent de la nourriture (produits à métaboliser) et de l'air et viennent donc constituer une culture fixée au voisinage des fibres 3. Les produits formés par la fermentation ou réaction biologique passent ensuite à l'intérieur des fibres 4 dont la porosité est choisie pour ne laisser passer que les produits désirés. Ceci constitue un effluent 8 qui quitte l'ensemble composite de membranes de filtration. Le reste des produits de la fermentation constitue un flux 9 qui est recyclé de façon interne pour balayer continuellement la culture fixée de microorganismes en vue de la nettoyer continuellement et de la débarrasser des microorganismes morts ou inactifs.

La figure 2 représente une autre disposition des trois types de membranes de la présente invention et, dans ce cas là, on a supposé que la réaction biologique était une fermentation anaérobie et les fibres de perméation gazeuse sont alors utilisées pour éliminer les gaz produits.

L'ensemble 10 se compose d'une première fibre creuse 12 entourée d'un écarteur 15, qui laisse passer vers l'extérieur les produits que les microorganismes doivent transporter. La troisième membrane est constituée par une série de fibres creuses 14 disposées au sommet d'un hexagone, chacune de ces fibres 14 étant entourée de trois fibres de perméation gazeuse 13 assurant l'élimination des gaz produits par le métabolisme des microorganismes qui viennent se fixer au voisinage des fibres 14 et 13. Le substrat à traiter 16 arrive à l'intérieur de la fibre 12, traverse partiellement cette membrane et subit une réaction biologique au niveau des microorganismes. Les produits recherchés sont recueillis par l'intermédiaire des fibres creuses 14 et quittent l'ensemble sous forme de l'effluent 18 tandis que les gaz produits par la réaction quittent l'ensemble par les fibres 13 sous forme de l'effluent 20.

Un recyclage interne 19 permet comme dans le cas précédent de balayer continuellement les cultures fixées des microorganismes.

Dans les deux cas de figure, pour que les filtrations à travers les différents types de membranes se fassent correctement, il faut que la pression de l'effluent à traiter 6 soit supérieure à la pression de l'effluent de recyclage interne 9 qui doit elle-même être supérieure à la pression de l'effluent 8 qui quitte l'ensemble. Les pressions peuvent être obtenues à l'aide de pompes placées dans les différents circuits, comme décrit ci-après.

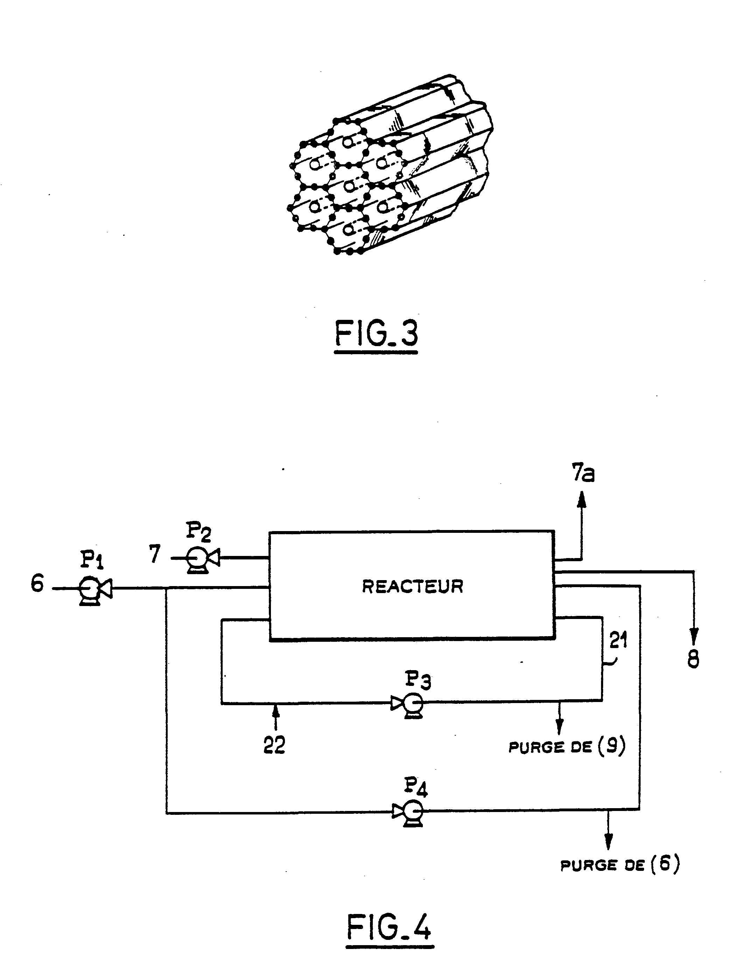

La configuration hexagonale de la série de fibres constituant la troisième membrane n'est bien entendu pas indispensable mais on a trouvé qu'elle facilite l'assemblage de plusieurs ensembles composites en un faisceau, chaque côté de l'hexagone étant commun à un autre hexagone (figure 3). Bien entendu d'autres dispositions et d'autres types d'assemblage peuvent être envisagés, pour autant que l'on y retrouve les trois types de membranes associés de la façon constituant l'ensemble composite de la présente invention.

De même, la disposition de la figure 1 a été représentée dans le cas d'une fermentation aérobie et celle de la figure 2 dans le cas d'une fermentation anaérobie. Il est évident que ceci pourrait être inversé, en modifiant simplement le sens de filtration des fibres de perméation gazeuse (sens centrifuge dans le cas d'une fermentation aérobie et sens centripète dans le cas d'une fermentation anaérobie).

L'ensemble ou les faisceaux d'ensembles composites selon la présente invention peuvent s'utiliser dans un bioréacteur tel que celui représenté schématiquement sur la figure 4. La description de ce réacteur sera faite en considérant qu'il contient des ensembles composites de membranes de filtration tel que représenté sur la figure 1, en vue d'une fermentation aérobie, et les numéros de référence identiques désignent des éléments identiques. L'effluent à traiter 6 est introduit dans le réacteur dans lequel sont disposés des ensembles composites selon la figure 1. Une pompe $P_1$ assure une pression suffisante du substrat 6 à l'intérieur de chacune des fibres 2 dans le réacteur. De l'air 7 est introduit par l'intermédiaire d'une pompe $P_2$ à l'intérieur des fibres de perméation gazeuse 3, l'excès d'air sortant du réacteur sous forme d'effluent gazeux 7a. Le produit recherché sort du réacteur sous forme de l'effluent 8. La partie de substrat 6 qui traverse le réacteur sans subir de traitement est recyclée par l'intermédiaire de la pompe $P_4$ et renvoyée au réacteur avec le substrat frais qui arrive. Une purge est prévue dans le circuit de recyclage afin d'éliminer l'excès de condensat non filtrable au travers de la membrane 2.

Ainsi qu'il a été indiqué précédemment, un recyclage interne est prévu pour l'effluent 9. Cet effluent qui se compose de substrat partiellement traité et débarrassé des produits recherchés est recyclé par l'intermédiaire d'une conduite 21 sur laquelle est placée une pompe $P_3$ assurant le recyclage interne de l'effluent 9. Ce recyclage interne, sans aucun contact avec le substrat à traiter ou l'effluent produit, permet l'introduction des microorganismes par exemple en 22, ces microorganismes venant éventuellement se fixer dans les ensembles composites à l'endroit où ils trouveront leur nourriture. Une purge est également prévue dans ce circuit de recyclage interne afin d'éliminer l'excès de courant 9. Les pompes $P_1$ et $P_3$, ainsi que la pompe $P_4$ assurant le recyclage de substrat non traité 6 sont réglées de façon à donner les pressions désirées dans le réacteur.

Les ensembles composites de membranes de filtration, le bioréacteur à membranes de filtration et le procédé de traitement biologique et de filtration simultanée selon la présente invention ont de larges applications dans différents domaines, et on ne citera qu'à titre d'exemple les domaines alimentaires, pharmaceutiques ainsi que le traitement des eaux. On verra en outre que plusieurs réacteurs effectuant des réactions biologiques identiques ou distinctes peuvent être utilisés successivement, de même que l'on pourrait séparer plusieurs zones de réactions distinctes à l'intérieur d'un même réacteur en modifiant les caractéristiques des ensembles composites dans chacune des zones distinctes.

## Revendications

1. Ensemble composite ordonné de membranes de filtration permettant une réaction biologique (ou fermentation) et une filtration simultanée des produits de la réaction, caractérisé en ce qu'il comprend :
   - une première membrane constituée d'une ou d'un faisceau de fibres creuses (2, 12) amenant le substrat à traiter et laissant passer de façon centrifuge les produits sur lesquels doit se faire la réaction biologique,
   - une seconde membrane discontinue et constituée d'une série de fibres (3, 13) de perméation gazeuse, disposées autour de la première membrane, cette seconde membrane assurant les échanges gazeux de la réaction biologique et
   - une troisième membrane discontinue et constituée d'une série de fibres creuses (4, 14), situées au voisinage des fibres de perméation gazeuse de la seconde membrane et laissant passer de façon centripète les produits recherchés de la réaction biologique résultant de la culture de microorganismes établie dans l'espace intermembranaire existant entre les trois types de membrane, les membranes étant séparées par des écarteurs (5, 15) qui permettent le passage des différents flux entre les membranes et les pressions de ces différents flux étant choisies de façon appropriée.

2. Ensemble composite ordonné selon la revendication 1, caractérisé en ce que les trois membranes sont coaxiales, la troisième membrane (1) entourant la seconde (3) qui entoure la première (2).

3. Ensemble composite ordonné selon la revendication 1, caractérisé en ce que la deuxième membrane est constituée de plusieurs ensembles de fibres (13), chaque ensemble étant disposé autour d'une des fibres (14) de la troisième membrane.

4. Ensemble composite ordonné selon l'une quelconque des revendications 1 à 3, caractérisé en ce que les fibres de perméation gazeuse (3) laissent passer les gaz de façon centrifuge dans le cas d'une fermentation aérobie.

5. Ensemble composite ordonné selon l'une quelconque des revendications 1 à 3, caractérisé en ce que les fibres de perméation gazeuse (13) laissent passer les gaz de façon centripète dans le cas d'une fermentation anaérobie.

6. Ensemble composite ordonné selon l'une quelconque des revendications 1 à 5, caractérisé en ce que les fibres (4, 14) de la troisième membrane sont placées aux sommets et sur les côtés d'un hexagone permettant la réunion de plusieurs ensembles en un faisceau ordonné de fibres.

7. Bioréacteur à membrane de filtration, comportant une arrivée de substrat à traiter (6), éventuellement une arrivée de gaz (7), une sortie d'effluent gazeux (7a), une sortie d'effluent de produits désirés (8) et un circuit de recyclage du substrat non traité, caractérisé en ce que le réacteur comporte un ou plusieurs ensembles composites ordonnés de membranes de filtration selon l'une quelconque des revendications 1 à 6, et un circuit de recyclage interne (21) de l'effluent traité débarrassé des produits recherchés.

8. Bioréacteur à membrane de filtration selon la revendication 7, caractérisé en ce que le circuit de recyclage interne (21) comporte une entrée (22) permettant l'introduction des microorganismes dans le réacteur.

9. Bioréacteur à membrane de filtration selon l'une quelconque des revendications 7 et 8, caractérisé en ce que les deux circuits de recyclage comportent des purges.

10. Procédé de traitement biologique et filtration simultanés, dans lequel on amène un substrat à traiter sur des microorganismes fixés et on filtre le produit fermenté pour séparer les produits recherchés de l'effluent à rejeter, caractérisé en ce qu'on introduit le substrat à traiter dans un réacteur contenant un ensemble ou un faisceau d'ensembles composites ordonnés de membranes de filtration selon l'une quelconque des revendications 1 à 6, le substrat arrivant à l'intérieur de la ou des fibres creuses constituant la première membrane, on établit une culture de microorganismes dans l'espace intermembranaire existant entre les trois types de membranes, et on recueille les produits désirés par l'intermédiaire des fibres constituant la troisième membrane.

11. Procédé selon la revendication 10, caractérisé en ce qu'on établit un recyclage interne du substrat partiellement traité.


**Claims**

1. Composite ordered assembly of filter membranes to permit biological reaction (or fermentation) and simultaneous filtration of the reaction products, characterized in that it comprises :
- a first membrane consisting of one or more bundles of hollow fibres (2, 12) guiding the substrate to be treated and permitting the centrifugal passage of the products on which the biological reaction is to take place ;
- a second discontinuous membrane, consisting of a set of gas-permeable fibres (3, 13) arranged around the first membrane, this second membrane permitting the gas exchanges for the biological reaction ; and
- a third discontinuous membrane consisting of a set of hollow fibres (4, 14) located in the vicinity of the gas-permeable fibres of the second membrane and allowing the centripetal passage of the required products of the biological reaction resulting from the culture of micro-organisms established in the inter-membrane space present between the three types of membranes, the membranes being separated by spacers (5,15) which permit the passage of the different flows between the membranes, the pressures of these different flows being selected in a suitable way.

2. Composite ordered assembly according to Claim 1, characterized in that the three membranes are coaxial, the third membrane (1) surrounding the second (3) which surrounds the first (2).

3. Composite ordered assembly according to Claim 1, characterized in that the second membrane consists of a number of sets of fibres (13), each set being arranged around one of the fibres (14) of the third membrane.

4. Composite ordered assembly according to any of Claims 1 to 3, characterized in that the gas-permeable fibres (3) allow the centrifugal passage of gases in the case of aerobic fermentation.

5. Composite ordered assembly according to any of Claims 1 to 3, characterized in that the gas-permeable fibres (13) allow the centripetal passage of gases in the case of anaerobic fermentation.

6. Composite ordered assembly according to any of Claims 1 to 5, characterized in that the fibres (4,14) of the third membrane are placed at the vertices and on the sides of a hexagon, enabling a number of sets to be combined in an ordered bundle of fibres.

7. Filter membrane bioreactor, comprising an inlet for the substrate to be treated (6), a gas inlet if necessary (7), a gaseous effluent outlet (7a), an outlet for the effluent of required products (8) and a recycling circuit for the untreated substrate, characterized in that the reactor comprises one or more composite ordered assemblies of filter membranes according to any of Claims 1 to 6, and an internal recycling circuit (21) for the treated effluent from which the required products have been removed.

8. Filter membrane bioreactor according to Claim 7, characterized in that the internal recycling circuit (21) comprises an inlet (22) allowing micro-organisms to be introduced into the reactor.

9. Filter membrane bioreactor according to either of Claims 7 or 8, characterized in that the two recycling circuits include drains.

10. Procedure of biological treatment and simultaneous filtration, in which a substrate to be treated is added to fixed micro-organisms and the fermented product is filtered to separate the required products from the effluent to be discharged, characterized in that the substrate to be treated is introduced into a reactor containing an assembly or a bundle of ordered composite assemblies of filter membranes according to any of Claims 1 to 6, the substrate entering the interior of the hollow fibre or fibres constituting the first membrane ; a culture of micro-organisms is established in the inter-membrane space between the three types of membranes ; and the required products are collected through the fibres constituting the third membrane.

11. Procedure according to Claim 10, characterized in that internal recycling of the partially treated substrate is established.

## Patentansprüche

1. Geordnete und zusammengesetzte Filtrationsmembraneinheit, welche eine biologische Reaktion (oder Fermentation) und eine gleichzeitige Filtration der Reaktionsprodukte ermöglicht, dadurch gekennzeichnet, daß sie umfaßt :
- eine aus einer Hohlfaser oder einem Bündel von Hohlfasern (2, 12) bestehende erste Membran, welche das zu behandelnde Substrat aufnimmt und zentrifugal die Produkte passieren läßt, mit welchen die biologische Reaktion stattfinden soll,
- eine diskontinuierliche und von einer Serie von gasdurchlässigen Fasern (3, 13) gebildete zweite Membran, welche Fasern um die erste Membran angeordnet sind, wobei die zweite Membran den Gasaustausch der biologischen Reaktion sicherstellt, und
- eine diskontinuierliche und von einer Serie von Hohlfasern (4, 14) gebildete dritte Membran, welche Fasern nahe den gasdurchlässigen Fasern der zweiten Membran angeordnet sind und zentripetal die in der biologischen Reaktion, welche aus der im zwischen den Membranen bestehenden Raum vorgesehenen Mikroorganismuskultur resultiert, gesuchten Produkte hindurchtreten läßt, wobei die Membranen durch Abstandshalter (5, 15) getrennt sind, die den Durchtritt von unterschiedlichen Flüssen zwischen den Membranen und auf geeignete Weise gewählte Drücke dieser unterschiedlichen Flüsse ermöglichen.

2. Geordnete und zusammengesetzte Einheit nach Anspruch 1, dadurch gekennzeichnet, daß die drei Membranen koaxial angeordnet sind, wobei die dritte Membran (4) die die erste Membran (2) umgebende zweite Membran (3) umgibt.

3. Geordnete und zusammengesetzte Einheit nach Anspruch 1, dadurch gekennzeichnet, daß die zweite Membran von mehreren Einheiten von Fasern (13) gebildet wird, wobei jede Einheit um eine der Fasern (14) der dritten Membran angeordnet ist.

4. Geordnete und zusammengesetzte Einheit nach einem der Ansprüche 1 bis 3, dadurch gekennzeichnet, daß die gasdurchlässigen Fasern (3) im Fall einer aeroben Fermentation die Gase zentrifugal hindurchtreten lassen.

5. Geordnete und zusammengesetzte Einheit nach einem der Ansprüche 1 bis 3, dadurch gekennzeichnet, daß die gasdurchlässigen Fasern (13) im Fall einer anaeroben Fermentation die Gase zentripetal hindurchtreten lassen.

6. Geordnete und zusammengesetzte Einheit nach einem der Ansprüche 1 bis 5, dadurch gekennzeichnet, daß die Fasern (4, 14) der dritten Membran an den Scheiteln und Seiten eines Sechseck angeordnet sind, um die Vereinigung von mehreren Einheiten in ein geordnetes Bündel von Fasern zu ermöglichen.

7. Filtrationsmembran-Bioreaktor mit einem Zulauf (6) für zu behandelndes Substrat, gegebenenfalls einem Zulauf (7) für Gas, einem Ablauf (7a) für Gas, einem Ablauf (8) für die gewünschten Produkte und einem Rezyklierkreislauf für nicht behandeltes Substrat, dadurch gekennzeichnet, daß der Reaktor eine oder mehrere geordnete und zusammengesetzte Filtrationsmembraneinheiten nach einem der Ansprüche 1 bis 6 sowie einen internen Rezyklierkreislauf (21) für behandelten und von den gesuchten Produkten freie Abwasser-Effluenten umfaßt.

8. Filtrationsmembran-Bioreaktor nach Anspruch 7, dadurch gekennzeichnet, daß der interne Rezyklierkreislauf einen Eingang (22) aufweist, welcher die Zugabe von Mikroorganismen in den Reaktor ermöglicht.

9. Filtrationsmembran-Bioreaktor nach Anspruch 7 oder 8, dadurch gekennzeichnet, daß die zwei Rezyklierkreisläufe Entleerungen aufweisen.

10. Verfahren zur gleichzeitigen biologischen Behandlung und Filtration, wobei ein zu behandelndes Substrat befestigten Mikroorganismen zugeführt wird und das fermentierte Produkt zur Trennung der gesuchten Produkte vom abzuscheidenden Abwasser gefiltert wird, dadurch gekennzeichnet, daß das zu behandelnde Substrat in einen Reaktor eingebracht wird, welcher eine geordnete und zusammengesetzte Einheit oder ein Bündel von derartigen Einheiten von Filtrationsmembranen nach einem der Ansprüche 1 bis 6 enthält, daß das Substrat in das Innere über eine oder die die erste Membran bildende (n) Hohlfaser (n) eingebracht wird, daß eine Mikroorganismuskultur in dem zwischen den drei Membranarten bestehenden Zwischenmembranen aufgebaut wird und daß die gewünschten Produkte mit Hilfe der die dritte Membran bildenden Fasern gesammelt werden.

11. Verfahren nach Anspruch 10, dadurch gekennzeichnet, daß eine interne Rezyklierung für partiell behandeltes Substrat eingerichtet wird.

## FIG_1

## FIG_2

FIG.3

FIG.4